(19) Europäisches Patentamt
European Patent Office
Office européen des brevets

(11) Publication number: **0 003 679**
**A1**

(12) # EUROPEAN PATENT APPLICATION

(21) Application number: **79300193.4**

(22) Date of filing: **08.02.79**

(51) Int. Cl.²: **A 61 K 33/34**
// (A61K33/34, 33/32, 33/26, 33/14, 33/06, 31/525, 31/51, 31/455, 31/44, 31/375)

(30) Priority: **08.02.78 AU 3302/78**

(43) Date of publication of application: **22.08.79**
**Bulletin 79/17**

(84) Designated Contracting States: **BE DE FR GB IT LU NL**

(71) Applicant: **Neaverson, Michael Anthony, Dr., P.O. Box 566, Strathfield, N.S.W. 2135 (AU)**

(72) Inventor: **Neaverson, Michael Anthony, Dr., P.O. Box 566, Strathfield, N.S.W. 2135 (AU)**

(74) Representative: **Coxon, Philip, Eric Potter & Clarkson 14 Oxford Street, Nottingham NG1 5BP (GB)**

(54) **Body replacement therapy formula.**

(57) A composition for treatment of heat exhaustion or heat syncope, comprising measured or titrated amounts of essential electrolytes, elements, minerals and vitamins to replete body stores of such essential electrolytes, elements, minerals and vitamins required for normal sweat gland functions.

The composition may include potassium, iron, magnesium, manganese, chloride and sulphate ions, water-soluble vitamins and possibly sodium, calcium, copper and bicarbonate ions and carbohydrates.

EP 0 003 679 A1

# BODY REPLACEMENT THERAPY FORMULA

The present invention relates to a method of, and to compositions useful for, treatment of heat exhaustion, heat stress or heat syncope.

There are a number of variables which are important in the individual's response to the environment, including air temperature, humidity, radiant heat of the surroundings, and air speed, and these affect individuals in various ways. Other factors which are important in the individuals response to environment include rate and type of work, acclimatization of the individual, amount and type of clothing, age and sex, and state of nutrition.

One of the major problems facing emigrants into, or people working in, arid environments is their patho-physiological response to heat. This is particularly so in the case of persons required to do heavy manual labour in such environments. Men who are in good physical condition achieve acclimatization more quickly than those in poor condition. Additionally, during work they do not exhibit as high a physiological strain or suffer as much stress in the heat as their less fit colleagues.

With increasing availability of budget air fares, tourism is becoming a major industry with significant increments each year.

Based on 1974 statistics, over 8 million tourists leave temperate countries for an annual holiday in

tropical zones where they become subjected to unaccustomed heat stress.

This heat stress leads to an increase in morbidity and general fatigue and is accompanied by failure to gain any benefit from their relaxation periods.

Due to increased mobility associated with modern means of transportation, men serving in the armed forces are particularly likely to suffer from significant heat stress, particularly where they are confined to small rooms under desert conditions ... for example, in tanks, aeroplanes, engine rooms etc. An important aspect of the availability of armed forces in the latter half of the 20th century is the ability to move numbers of men from one climatic conditions to another with great speed. So as to ensure such mobility of armed forces who may be required to become operational in tropical regions at short notice, it is important to increase the speed of acclimatization as far as possible.

With the increase in preventative medicine considerable medical education has been devoted to the interest to maintain physical fitness. This has resulted in a number of young/middle aged men and women undertaking fairly strenuous physical activity which is associated with considerable sweat loss particularly in the obese. The increase in active participation in sports particularly athletics and football create an additional group of people who may be predisposed towards the symptoms associated with hypokalaemia. The high incidence of mortality and considerable morbidity amongst the elderly during the occasional heat waves which occur from time to time in major cities presents a serious problem. Major causes of death amongst these people is associated with the dangerous hypokalaemia resulting from excessive sweat loss. The lowering of potassium levels is particularly dangerous in patients taking cardiac glycosides and diuretic preparations.

Many elderly people require these preparations for the control of cardiac symptoms which are often given without adequate potassium supplementation and therefore losses of potassium and sweat during the heat wave may predispose to fatal dysrhythmias contributing significantly to the mortality associated with heat exhaustion.

It has been found that to successfully treat or prevent heat exhaustion or heat syncope it is essential to provide a suitably balanced formulation which, in combination, replenish the essential electrolytes, minerals and vitamins lost from the body as a result of sweating. Replacement of sodium and chloride alone does not replenish other losses of essential electrolytes and vitamins; an excessive intake of replacement salts may be associated with adverse effects and death.

Sodium chloride tablets are often not absorbed and injudicious use may be dangerous. It has been found that the ideal replacement is either a liquid preparation or granules for reconstitution containing a titrated dose of each of the essential electrolytes, elements and vitamins, which, taken prophylactically, produce a synergistic effect in treatment of heat syncope, result in repletion of body stores and ensure maximum efficiency of body function.

It is an object of this invention to provide specifically formulated compositions which will prevent and alleviate symptoms associated with both acute and chronic heat stress or heat exhaustion, and to enable people in hot environments to better tolerate or to perform work under trying conditions.

It is a further object of this invention to provide specially formulated compositions to supplement the diet of people during periods of acclimatization in hot environments whereby a reduction in the time to acclimatize individuals can be realised.

The present invention provides compositions for treatment of heat exhaustion or heat syncope comprising

measured or titrated amounts of essential electrolytes, elements, minerals and vitamins to replete body stores of such essential electrolytes or minerals and vitamins required for normal sweat gland function.

More specifically, the present invention provides compositions for treatment of heat exhaustion or heat syncope comprising measured or titrated amounts of Potassium, Iron, Magnesium, Manganese, Chloride, Sulphate and water-soluble vitamins, optionally the compositions may further comprise measured or titrated amounts of Sodium, Calcium, Copper, Bicarbonate, a caloric source or carbohydrate, together with other minor constituents or excipients to supplement or replace other ingredients of the composition.

The invention also provides a method of treatment, or prevention, of heat exhaustion or heat syncope by treating persons with the above described compositions.

The compositions according to the present invention may be presented in three forms, the first form for use by people who undertake light work for protracted periods under conditions of excessive environmental temperatures, the second form for use by people who undertake heavy physical activity under conditions of excessive environmental temperatures for protracted periods and the third form is for use by people who are exposed to high environmental temperatures for relatively short periods of time (e.g. tourists to tropical areas) or to people undertaking sedentary work under conditions of high environmental temperatures. As the compositions of the present invention provide a balanced solution of essential electrolytes, elements and vitamins, the user is protected against potential overdosage which may occur with solid sodium chloride preparations.

Heat is a stress to which most people are exposed at one time or another. A large proportion of the world's

population lives and works in the tropical zones, where the climatic conditions vary from the hot, dry, desert climate to the hot, humid, jungle type of climate, and where the main factors which modify these conditions are the variability in the shapes of the land masses, the altitude of the areas concerned and the direction of the winds that blow over them. The climatic heat stress presented by the desert conditions take the form of large heat gains by convection and particularly by radiation. In the hot, humid jungle type of climate the stress is due rather to the restriction imposed by the environment on the evaporation of sweat. Thus, although the total heat stress in the two types of climate may sometimes be similar, the threat to thermoregulation may depend on different principles in the two situations.

While most tropical areas have a continuous, albeit a varying heat stress throughout the year, there are many other areas in the temperate climatic zones where an intermittent seasonal heat stress can be found. In addition there are few places that are not subject to heat waves; these heat waves, which often occur unexpectedly in otherwise equable climates, may present relatively high levels of heat stress since the inhabitants of such places have no previous opportunity to develop the ameliorating physiological adaptations that we know as acclimatization

In addition to naturally occurring hot climates in different geographical locations, there are many situations in industry or in the armed forces where "artificial" hot climates may be found. These artificial climates may be the result of, or may even be deliberately created for, some industrial process. For instance, there are often hot working conditions in mines partly because of the increase of temperature with depth and partly because of the difficulty of ventilation; in the cotton industry, warm moist atmospheres are maintained

because these conditions have been considered best for cotton weaving. Further examples may be found in the steel, glass, rubber or other industries. In the armed forces rather special problems exist, often associated with small, enclosed spaces that may be difficult to ventilate or to aircondition. Such compartments may be found in ships in tropical waters or in tanks or in aircraft in the desert, or in fast aircraft where the surface of the machine heats up during flight, due to air friction.

For the most part, industrial work presents only low levels of heat stress on men but in a number of cases, the level of heat stress involved in both everyday working situations and in emergency conditions ( as in firefighting or maintenance work in some enclosed spaces) may be greater than any that can be found naturally. It is seldom realized, for instance, that there are many miners in continental Europe who are exposed underground daily to a climatic stress greater than can be found in all but the most extreme of the naturally occurring climates on the earth's surface.

Mild and severe heat casualities have been reported from both naturally occurring and artificial climates. For the most part, the incidence of heat stroke and particularly of death due to heat receives more prominence in the literature than any other heat disorder; reports range from an increase in the occurrence of deaths during heat waves in large cities like London and New York, where the normal climatic heat stress is relatively low, to deaths in natural or in artificial climates where the level of heat stress is very high. Although the incidence of less severe heat disorders is given little prominence, they occur with much greater frequency.

There is little doubt that heat disorders most frequently occur for one or more of three reasons;

(1) The existence of factors such as some measure of dehydration or the lack of acclimatization, which predispose to these disorders; (2) the lack of proper appreciation of the dangers involved in exposure to heat either on the part of the supervising authority or of the individuals at risk; and (3) accidental or unforeseeable circumstances which lead to the exposure of men to very high heat stress. Despite the efficient thermoregulatory system with which man is endowed, it is relatively easy to induce failure in it by neglect, and it is this neglect that must be held responsible for the majority of deaths.

An increase in external temperature is followed by an increase in body temperatures. This rise in body temperature is followed by a reflex peripheral vasodilation and an increase in sweat production by the sweat glands. Sweat is a watery hypotonic solution containing many solutes. Evaporation of the sweat allows cooling of the skin and dissipation of the heat, and is the body's main defence against heat stress.

Maximum rates of sweating which cannot be maintained for long periods are of the order of 2 litres per hour. After exposure to a hot environment for a period of days the capacity to produce sweat increases. This process by which the body adapts to increased heat stress is referred to as acclimatization. Well acclimatized men may produce up to 3 litres of sweat per hour for short periods which may present a threat to homeostasis.

A number of days exposure is required to enable the body to undertake the necessary changes, which include reduction in deep body temperature and increased sweat volume, with decreased mineral content and reduced urine output.

During the acclimatization the concentration of a number of minerals and vitamins in the body are reduced to levels below those which can be explained by sweat loss

alone. It appears that these substances are essential for normal sweat gland function.

It has been demonstrated that thermal sweating after heat acclimatization increases from 0.48 to 0.70 mg per $cm^2$ of surface area. The increased sweat output is primarily due to increased glandular activity at the level of the sweat glands.

It has also been reported that while sweat production increases by some 30% during acclimatization, the increase in volume of sweat evaporated is only of the order of 10%. This overproduction of sweat during the early stages of exposure is considered detrimental having no advantage for thermo-regulation and posing severe disadvantages for body fluid control. Small men, because of their relatively small surface area are considerably disadvantaged when working in hot environments.

The changes which occur during the process of acclimatization may be summarised as follows;

(i) Increase in body temperature and pulse rate.

(ii) 30% increase in sweat production.

(iii) 10% increase in sweat evaporation.

(iv) Reduction in urine volume,

(v) Decrease in sodium excretion in sweat and urine.

(vi) Potassium excretion remains unchanged.

(vii) A reduction in the sodium potassium ratio from 15 to 1 and 5 to 1.

(viii) Reduction in plasma ascorbic acid levels.

(ix) Reduction in body temperature and pulse rate.

The medical information concerning the effects on the individual undergoing acclimatization or relocation to a more arid environment clearly demonstrates that such people can be seriously affected in the short and long term by fluid and electrolyte loss due to excessive production of sweat.

Sodium Chloride, Potassium and water are the main

components of sweat. However, the sweat glands also secrete other water soluble minerals and vitamins. Among the most important are Magnesium, Iron and Vitamin C.

The effects of exposure to heat and the resultant excessive sweat loss range from mild discomfort, lassitude and a reduction in the ability to perform work to exhaustion and even death.

It has been estimated that insensible water losses from the skin and lungs of the average adult in temperate climates amounts to about 1 litre daily; in warm surroundings the insensible loss may be twice this amount or more. If sweating is present, insensible loss from the skin need no longer be considered. In practice the total evaporative fluid loss for the skin and lungs is measured over a selected unit of time and the observed amount is called the sweat loss.

The amount of sweat loss per hour rises with the temperature and with the type of activity. The Table below illustrates the increase in sweat loss during moderately heavy work (300 kcal/hr) light work (210 kcal/hr) and sedentary work (120 kcal/hr) under varying air temperature conditions.

| | TEMPERATURE | | |
|---|---|---|---|
| | 32°C | 38°C | 43°C |
| Moderately heavy work | 0.75 | 1.0 | 1.5 |
| Light work | 0.40 | 0.8 | 1.0 |
| Sedentary | 0.10 | 0.3 | 0.55 |

TABLE

Sweat loss in litres per hour at various external temperatures and activities.

The intial effect of water depletion is to cause a

diminution in the extracellular fluid volume. Consequent upon this fluid moves from the intracellular space to the extracellular compartment with intracellular depletion.

There are three clinical grades of water depletion; (i) Mild - a deficit of approximately 2% body weight equivalent to a loss of 1.5 litres. This is accompanied by a reduction in urine output and increased urinary loss of potassium. The only symptcm is mild thirst.
(ii) Moderate - a deficit of 6% body weight equivalent to a fluid loss of 4.2 litres. Symptoms are increasing thirst, tachycardia, hypotension, general feeling of fatique and foreboding and elevation of body temperature.
(iii) Severe - a deficit of 7% body weight. Reduction in mental activity, delerium, coma and death.

It is known that eccrine sweating is an extremely efficient mechanism for heat dissipation in men where secretions of 10 to 12 litres per day represent an evaporative heat loss of 5,800 to 7,000 kcals.

If the sodium concentration in the sweat was not modified during the period of acclimatization, secretion of this volume of sweat would amount to a loss of 30 Gms sodium chloride per day.

The initial effect of sodium depletion is to cause a fall in the osmolarity of the extracellular space. The body reacts to this by excreting water through the kidney therefore incurring a reduction in the volume of the extracellular space until there is a progressive fall in circulatory volume.

Salt depletion is characterised by fatique, nausea, vomiting, giddiness, hypotension and circulatory failure.

Symptoms may be graded into:

| | | | |
|---|---|---|---|
| (i) | Early | loss 0.5 Gms/Kgbw | Fatigue, giddy |
| (ii) | Moderate | loss 0.5 Gms/Kgbw | Nausea, Cramps Vomiting |
| (iii) | Severe | loss 7.0 GMs/Kgbw | Hypotension, Shock |

Prevention of salt depletion can be effected by an adequate controlled intake titrated to work load and external temperature. A reduction in intake can be effected after the period of acclimatization.

Excessive sodium loss from the body is, however, prevented by a reduction in concentration of sodium in both the sweat and urine due to an increase in aldosterone secretion.

Aldosterone is the principle mineralo-corticoid of the body and influences the rate of sodium and potassium transport across cell membranes. Of particualr importance in acclimatization is the rate of transport across renal tubule and sweat glands.

Increase in aldosterone causes retention of sodium and excretion of potassium.

On a normal diet (150 to 250 mEq sodium/day) secretion of aldosterone is approximately 100 mcg/day. Severe sodium depletion will increase this four to fivefold.

Aldosterone secretion is subject to a diurnal rhythm increasing during the day when the patient is up and about and decreasing during rest at night.

Increased aldosterone activity is, however, not a prerequisite of the acclimatization process unless a negative salt balance is first established. Europeans who performed light duties and maintained a generous salt and water intake during early exposure in the Persian Gulf did not increase their urinary aldosterone levels.

The process of acclimatization ensures an 80% improvement in salt conservation and temperature regulation occurring within one week of exposure. During the early stages of acclimatization it is, however, the loss of salt and water which is most likely to seriously affect homeostasis.

While modification of sodium excretion occurs during

acclimatization no such reduction occurs in potassium excretion. For instance it is known that the concentration of potassium in sweat is twice that of the plasma, i.e. an average of 9.4 mEq.

Men undertaking basic military training may secrete 12 litres of sweat per day with a normal loss of potassium in excess of 100 mEq, which is often higher than the dietetic intake. Fatalities from heat stroke suffered by healthy, young military recruits are numerous and well documented. Such reports indicate that potassium loss plays a key role in acute heat exhaustion.

Unlike sodium, potassium excretion in the sweat is proportional to the sweating rate as no significant reabsorption occurs. Daily potassium loss by this route in men working for eight hours in hot surroundings may therefore be significant and comparable to losses in chronic diarrhoea.

A fall of serum potassium occurs during severe work or exercise in heat. Exercise causes increased respiratory exertion with consequent respiratory alkalosis allowing a movement of potassium ions into the intracellular compartment.

Potassium loss lowers muscle efficiency and increases the risk of muscle injury, the heart muscle in particular is affected resulting in reduced function and lowered work efficiency. Hence the importance of Potassium in maintaining bodily function during short and long term exposure to heat is probably underestimated, the popular lay view being that the effects of heat exposure can be offset by the intake of additional salt with the necessary water. In fact, during heat stress, salt ingestion can increase potassium excretion.

About half the patients with acute heatstroke are found to be hypokalaemic and a reduction in total body potassium may be of aetiological importance in the genesis of heatstroke.

Whilst sodium, chloride, potassium and water are the

main components of sweat, other water soluble minerals and vitamins are lost particularly during the secretion of large volumes of sweat during heavy work.

Men working in extreme heat may lose over 100 mg calcium per hour. Whilst sweat calcium levels show individual variation a consensus of opinion reveals an average value of 2.6 mEq. Other values are 0.3 + 0.12 mEq, 1.37 to 2.6 Mg%, 1-8 mg%, 3-30 mg%. The concentration of calcium in the sweat equates with the ionised calcium concentration in the blood. Reduction in the ionized level of calcium produces over-excitability of nerves and muscles.

Sweat magnesium levels range from 0.13 to 0.99 mEq, and unacclimatized subjects exposed to arid conditions demonstrate a fall in serum magnesium levels. Deficiency of magnesium may occur under the same conditions that lead to a lack of potassium.

Deficiency of magnesium leads to depression, muscular weakness, vertigo and convulsions together with low voltage ECG change.

Sweat loss of copper may exceed 1 mg per day and this could result in a negative balance if sweat loss is high and copper intake is low. Copper deficiency may cause diarrhoea and anaemia.

Iron loss in sweat appears to be proportional to the body stores of iron. Healthy patients secrete 1.6 mgs per litre whereas iron-depleted patients secrete 0.36 mgs per litres.

Iron deficiency is endemic in most tropical countries partly due to inadequate dietary intake and partly due to parasitic infestation.

Whilst iron loss in the sweat in temperate areas is not significant, the marked increase in sweat production associated with exposure to high environmental temperatures may lead to a significant loss.

Iron depletion occurs far more frequently in tropical arid zones than temperate areas for the following reasons;

i.) The diet contains more cereals and phytates which form insoluble complexes in the bowel with the iron and prevent absorption.

ii.) Bleeding from the bowel is commonly due to parasiticinfestation e.g. hookworm.

iii) Haemolytic diseases e.g malaria are common in tropical area.

iv) Associated with the need to produce sweat significant losses of iron occur.

Reduction in the total body Iron results in reduced manual and physical capabilities which may be corrected by repletion.

Iron depletion may lead to anaemia but even in the absence of anaemia the Iron deficiency produces significant symptomatology.

A number of studies have shown that Iron deficient subject are less attentive and have less ability to concentrate.

A number of workers have shown that in physical activities patients who are Iron depleted or anaemic are unable to compete physically with those who are not. In an Indonesian study the work output of labourers in a rubber plantation was assessed and comparison made of the work done in a period of time by anaemic and non-anaemic workers.

As Iron is essential for adequate oxygen utilisation and normal muscle activity it was not surprising to find that the anaemic workers were significantly less efficient than their non-anaemic colleagues

The secretion of large volumes of sweat may be accompanied by the loss from the body of water soluble vitamins.

Whilst the loss of vitamins in sweat is small, such

losses become significant as sweat secretion increases; it appears that some vitamin are utilized in increased quantities in the process of sweating and therefore acclimatization.

For example, a study of the ascorbic acid levels of European residents in the tropics showed a significant fall in the levels of white cell ascorbic acid in men who had been resident for more than 4 months. In fact, there has been shown to be a significant decrease in the total circulating ascorbic acid occurring during the process of acclimatization. Further it is known that by supplementing patients with ascorbic acid during the period of acclimatization a significant beneficial effect occurred in the ability to work in the heat. Additionally, supplementation allows for a reduction in acclimatization time.

The clinical picture of ascorbic acid deficiency is characterised with haemorrhages resulting from the slightest trauma. Bleeding can occur under the skin and can be seen as bluish patches called "Ecchymosis". Haemorrhages can occur in the eyes, brain, nose, gastrointestinal tract or genitourinary tract. Haemorrhages occur near bones, in the sub-periosteal region and joints, causing swollen painful joints.

Reduction in ascorbic levels has been shown to be associated with a sub-clinical type of Scurvy in which the symptoms are associated with the musculo-skeletal system.

Vitamin C is beneficial in the treatment of a skin condition, prickly heat, which is associated with a malfunction of the sweat glands particularly in the tropics. Indeed, many workers in the tropics have been shown to have significant lower levels of ascorbic acid in their bodies than workers in temperate climates.

Maintaining the ascorbic acid concentration in the blood during acclimatization has been associated with a

decrease in deep body temperature and a maintenance of plasma volume. When volunteers were exposed to heat stress this increase in plasma volume was associated with a reduction in the total sweat output required to maintain a given deep body temperature.

In summary the ascorbic acid supplementation increases the rate of acclimatization and appears to improve the body's ability to withdstand high external temperatures.

Whilst a controversy continues over the amount of ascorbic acid lost in sweat, recent data clearly demonstrates the importance of adequate supplementation in the process of acclimatization.

Significant amounts of the B group of vitamins can be lost in sweat. Symptoms of Vitamin B deficiency include irritability and depression. It is well known that these conditions commonly affect people working in high temperatures.

Approximately 45 mcg per litre of Thiamine are lost in sweat therefore in workers undertaking heavy manual exertion in the tropics a daily loss of 0.4 mg per day could be anticipated. Dietetic intake in the tropics may be below this figure and, therefore, it would be prudent to supplement the intake of workers in order to ensure that carbohydrate metabolism maintains its normal efficiency.

Approximately 43 mcgs of Riboflavine (B2) may be lost in each litre of sweat secreted. Riboflavine plays an important role in maintaining an adequate respiratory function.

The process of respiration is essential for the normal oxygenation of red blood cells and is important in maintaining normal work output of the musculo-skeletal system. A deficiency of Riboflavine may be associated with a reduction in the efficiency of the system.

With high sweat loss of Riboflavine, supplementation

in manual workers or those exposed to high external temperatures is mandatory.

Losses of Pyridoxine (B6) in the sweat are similar to those of thiamine and riboflavine (40 mcg per litre). Consequently, similar losses may occur in heavy manual work which may lead to the development of microcytic anaemia. The presence of anaemia has been shown previously to be associated with a reduced work output. Supplementation of the diet of manual workers is therefore recommended.

Nicotinic acid appears in higher concentrations in the sweat than the other water soluble vitamins (140 mcg per litre).

In tropical areas dietary intake may be inadequate and as Nicotinic acid plays an important role in normal body metabolism, supplementation may be given to manual labourers in hot environments.

<u>SUMMARY</u>

1. With secretion of large volumes of sweat significant amounts of water soluble vitamins may be lost from the body.
2. Persons working in tropical areas have been shown to have decreased tissue level of ascorbic acid.
3. Man is unable to synthesize Vitamin C and depend upon adequate intake to maintain normal tissue levels.
4. Vitamin C supplementation has been shown to improve acclimatization rate and ability to work in the heat.
5. Vitamin B losses in sweat may be significant where sweat secretion rates are high.
6. Heavy manual workers require high caloric intake a large percentage of which may be provided by carbohydrates. Vitamin $B_1$ is required to ensure adequate metabolism of carbohydrates.
7. Vitamin $B_6$ is necessary for normal amino acid metabolism. Losses of this vitamin in sweat leads to significant reduction in body stores thereby threatening normal metabolism.

8. Vitamin $B_2$ is an essential vitamin for normal respiratory function. Significant losses in tropical areas may occur.

9. Nicotinic acid is necessary for normal protein metabolism and high concentrations of this vitamin are lost in sweat.

10. Supplementation with adequate levels of Vitamins B and C are necessary for all tropical residents, especially those who are long term residents or, alternatively, those undertaking heavy physical exertion.

Heavy work in severe heat may provoke a sweat loss of considerably more than one litre an hour but so high a sweat rate cannot be maintained for long periods and conditions become intolerable for some men after three to four hours, particularly if their electrolyte balance is affected.

There is a need to provide compositions useful for fluid, electrolyte and vitamin replenishment in people subjected to short term and long term heat stress. The importance of Potassium, Magnesium, Vitamin C and Iron to such people is specifically highlighted. People at risk may be broadly classified as follows;

<u>Chronic Heat Stress</u>

1. Those undertaking light work under conditions of high temperature such as sedentary workers in the Middle East and tropic zones, in Latin America and Southeast Asia.

<u>Acute Short-Term Heat Stress</u>

2. Those experiencing short term acute heat stress due to, for example, heat wave conditions, the early phase of acclimatization, visitors to hot climates, tourists, particularly the physically active. Military recruits undergoing basic training and /or relocation to hot climates, athletes, joggers etc.

Chronic Excessive Sweat Loss

3. Those undertaking heavy physical activity in a hot environment e.g. construction worker, quarriers in tropical zones together with miners, workers in steel foundries and other heavy industries in temperate zones.

According to the present invention, specific formulations containing measured amounts of minerals and vitamins has been designed for each of these categories.

CHRONIC HEAT STRESS

According to the invention there is provided a specific formulation, as an acclimatization aid, which contains titrated amounts of minerals and vitamins in a solid dose form. Each dose replaces the losses of essential minerals and vitamins which are likely to occur in one litre of sweat in acclimatized persons. Undertaking light work in tropical surroundings may result in the loss of two litres or more of sweat per day.

After acclimatization a person undertaking light, physical activity under conditions of high temperature does not require excess sodium chloride for the preservation of normal homeostasis.

The addition of supplemented sodium chloride under these conditions may be deleterious as by increasing urinary volume, an accentuation of potassium loss may readily occur.

It will be appreciated that it is still common practice for acclimatized people to supplement their intake by the use of salt tablets which, while not necessary in the majority of cases, may be dangerous.

In granulated form the formulation comprises the following ingredients per dose;-

| | |
|---|---|
| Potassium Chloride | 335 mg |
| Ascorbic Acid | 100 mg |
| Ferrous Sulphate | 10 mg |

| | |
|---|---|
| Magnesium Sulphate | 35 mg |
| Copper Sulphate | 0.2 mg |
| Manganese Sulphate | 0.2 mg |
| Thiamine | 0.5 mg |
| Riboflavine | 0.5 mg |
| Nicotinic Acid | 0.5 mg |
| Pyridoxine | 0.5 mg |

In tablet form the formulation comprises in addition per 600 mg tablet;-

| | |
|---|---|
| Avicel (microcrystalline cellulose) | 93.6 mg |
| PVP (Povidone 28-30) | 24 mg |

The tablets as well as the granulated form contain the essential minerals and vitamins required for normal sweat gland function.

Each tablet or the required dosage amount of the granulated form, includes titrated amounts of potassium, magnesium, manganese, copper, chloride sulphate and ascorbic acid together with other necessary constituents and excipients which combine to provide a total dosage weight of approximately 600 mgs (i.e. tablet form).

People working under sedentary conditions in tropical zones for protracted periods of time require up to three tablets per day in order to ensure adequate replenishment.

ACUTE SHORT-TERM HEAT STRESS

The second group of people are those experiencing acute short-term heat stress.

Under these conditions supplementation with the formulation described above for chronic heat stress may be insufficient to maintain normal bodily function. A second formulation has been designed to meet these additional requirements.

Acute heat stress may occur in a number of situations e.g. those in the early stages of acclimatization (3 to 5 days), exposed to heatwave conditions, visitors to hot climates whose degree of physical activity is at best

moderate but whose level of physical fitness is relatively low, elderly people in temperate cities exposed to heatwave conditions, and those who are undertaking considerable, and may be protracted physical activity with significantly greater sweat loss for short periods in temperate climates.

A very large number of tourists from temperate zones spend some weeks in hot climates each year.

Many of these people undertake increased physical activity during this time thereby become exposed to increased risk of heat stroke or heat exhaustion. The excessive sweat loss accompanied by the depletion of minerals and water soluble vitamins significantly contributes to these symptoms.

The consumption of alcoholic drinks as thirst quenchers when socialising, or perhaps to counteract the discomfort brought about by the hot environment, is common practice.

Alcohol increases the body's need for Vitamins B and C and also increases fluid output thereby magnifying the effects of heat stress.

There is considerable biochemical evidence that women taking oral contraceptives experience increased loss of minerals and vitamins B and C. As a result, many such women have reported symptoms of irritability and mental depression, which have been relieved after they were supplemented with the appropriate minerals and vitamins, in particular Pyridoxine ($B_6$) and Vitamin C. Women on oral contraception may be particularly vulnerable to heat stress.

Additional factors such as alcohol ingestion and/or taking of oral contraceptives increase the need for supplementation with an appropriate Vitamin-Mineral formulation. The second formulation has been designed to meet this need.

Footballers, male and female athletes/joggers, military personnel may secrete as much as 6 litres of sweat per day, and under such conditions, and in environments where heatstroke is likely to occur, the sweat loss of sodium may exceed the dietary intake. As in the early period of acclimatization or in acute heat stress where sweat loss may be excessive, sodium losses may be significant. For this reason granules of the second formulation contain added sodium.

Increased potassium loss in sweat occurs following increased physical activity and therefore the concentration of potassium in granules of the second formulation has been increased.

In order to ensure adequate absorption of sodium, and to provide a limited caloric source, sucrose has been added to the formulation. The granules are preferably flavoured and when dissolved in a glass of water provide a palatable and refreshing drink. The formulation is provided in sachet form in a recommended dose related to the degree of physical activity; usual dose in one sachet dissolved in a glass of water 2-4 times a day. One sachet is equivalent to a protracted sweat loss of one litre.

By use of this formulation to supplement the diet, the period required for acclimatization can be reduced, for example from 8 days to 4 days.

Granules of the second formulation contain titrated amounts of sodium, potassium, magnesium, manganese, bicarbonate, sulphate and chloride, with water, soluble vitamins including ascorbic acid, sucrose flavouring and other constituents and excipients, which when reconstituted with water presents a pleasantly flavoured refreshing drink. The second formulation comprises the following ingredients;-

| | |
|---|---|
| Sodium Chloride | 250 mg |
| Sodium Bicarbonate | 500 mg |

| | |
|---|---|
| Potassium Chloride | 745 mg |
| Calcium Gluconate | 250 mg |
| Magnesium Sulphate | 35 mg |
| Copper Sulphate | 0.2 mg |
| Manganese Sulphate | 0.2 mg |
| Ferrous Sulphate | 10 mg |
| Ascorbic Acid | 100 mg |
| Thiamine | 0.5 mg |
| Riboflavine | 0.5 mg |
| Pyridoxine | 0.5 mg |
| Nicotinic Acid | 0.5 mg |
| Sucrose | 7.5 mg |
| Citric Acid | 425 mg |

CHRONIC EXCESSIVE SWEAT LOSS

The third group of people at risk are those undertaking heavy protracted physical activity in a hot environment. Under these conditions sodium chloride losses may be excessive resulting in Sodium depletion. The added work done under heat dissemination from the body causes an increased requirement for caloric intake.

To meet these demands a third formulation is provided for repletion of body stores of essential electrolytes and vitamins. The addition of sucrose in a dose of 25 gms per litre provides the caloric source necessary for the evaporation of sweat.

Additional amounts of iron to compensate for added sweat losses replete iron stores in the body with consequent improvement in work output.

The ascorbic acid concentration has been reduced on a dose basis because of the increased daily consumption of this formulation. The third formulation is provided in granular form to be dissolved in the drinking water, to provide the following ingredients per litre;-

| | |
|---|---|
| Sodium Chloride | 500 mg |
| Sodium Bicarbonate | 1000 mg |

| | |
|---|---|
| Potassium Cholride | 745 mg |
| Calcium Gluconate | 250 mg |
| Magnesium Sulphate | 35 mg |
| Copper Sulphate | 0.2 mg |
| Manganese Sulphate | 0.2 mg |
| Ferrous Sulphate | 10 mg |
| Ascorbic Acid | 50 mg |
| Thiamine | 0.5 mg |
| Riboflavine | 0.5 mg |
| Pyridoxine | 0.5 mg |
| Nicotinic Acid | 0.5 mg |
| Sucrose | 25 Gms |
| Citric Acid | 850 mg |

All of the above formulations are prepared by conventional procedures used in the chemical and pharmaceutical industry. All components are individually pulverised and passed through a # 52 mesh sieve, weighed and thoroughly mixed. Granulation and tableting are carried out by conventional procedures.

Although the invention has been described above with reference to preferred features and to examples or preferred embodiments, it will be appreciated that the invention is in no way limited to specific examples or formulations, preferred features or embodiments, and that additions to, modifications of, or substitution in , the described compositions may be made without departing from the spirit or scope of the invention.

CLAIMS:

1. A composition for treatment of heat exhaustion or heat syncope, comprising measured or titrated amounts of essential electrolytes, elements, minerals and vitamins to replete body stores of such essential electrolytes, elements, minerals and vitamins required for normal sweat gland functions.

2. A composition according to Claim 1 comprising measured or titrated amounts of Pottasium, Iron, Magnesium, Manganese, Chloride, Sulphate and water-soluble vitamins.

3. A composition according to Claim 2 further comprising measured or titrated amounts of one or more of the group consisting of Sodium, Calcium, Copper, Bicarbonate and Carbohydrate.

4. A composition for treatment of heat exhaustion or heat stress comprising measured or titrated amounts of the following ingredients;-

Potassium Chloride
Ascorbic Acid
Ferrous Sulphate
Magnesium Sulphate
Copper Sulphate
Manganese Sulphate
Thiamine
Riboflavine
Nicotinic Acid
Pyridoxine

5. A composition according to Claim 4 in tablet form.

6. A composition according to Claim 5 comprising, per tablet dose, measured or titrated amounts of the following ingredients;-

KCl
Ascorbic Acid
$FeSO_4 7H_2O$
$MgSO_4 1H_2O$ (desiccated)

$CuSO_45H_20$
$MnSO_44H_20$
Thiamine HCl
Riboflavine
Nicotinic Acid
Pyridoxine
Avicel (microcrystalline cellulose)
PVP (Povidone 28-30)

7. A composition for treatment of heat exhaustion or heat stress comprising measured or titrated amounts of the following ingredients;-

Sodium Chloride
Sodium Bicarbonate
Potassium Chloride
Calcium Gluconate
Magnesium Sulphate
Copper Sulphate
Manganese Sulphate
Ferrous Sulphate
Ascorbic Acid
Thiamine
Riboflavine
Pyridoxine
Nicotinic Acid
Sucrose
Citric Acid

8. A composition according to any one of claims 1 to 7 substantially as hereinbefore described with reference to the examples.

9. A method of treatment of, or prevention of, heat exhaustion or heat syncope by treatment with a composition according to any one of Claims 1 to 8.

European Patent Office

# EUROPEAN SEARCH REPORT

0003679

Application number

EP 79 30 0193

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| X | MODERN DRUGS ENCYCLOPEDIA, 1961. "Dexatabs" | 1,7 | A 61 K 33/34// (A 61 K 33/34, 33/32, 33/26, 33/14, 33/06 31/525, 31/51, 31/455, 31/44 31/375) |
| | -- | | |
| X | UNLISTED DRUGS, 1976, page 180, "Sustalyte". | 1,7 | |
| | ---- | | |

TECHNICAL FIELDS SEARCHED (Int.Cl.²)

A 61 K 33/34
33/14

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 29-03-1979 | BRINKMANN |

EPO Form 1503.1 06.78